# EUROPEAN PATENT SPECIFICATION

(11) **EP 0 540 945 B1**
(45) Date of publication and mention of the grant of the patent: **18.03.1998**
(21) Application number: 92118053.5
(22) Date of filing: 22.10.1992
(51) Int. Cl.: A61K 35/10

(54) **Compositions comprising an isothiazole derivative and a peat-derived bioactive product**
Präparate enthaltend Isothiazol-Derivate und ein aus Torf gewonnenes bioaktives Produkt
Compositions contenant un dérivé d'isothiazole et un dérivé bioactif de tourbe

(30) Priority: 26.10.1991 EP 91118269; 28.09.1992 EP 92116552
(43) Date of publication of application: 12.05.1993
(73) Proprietor: Torf Establishment, FL-9490 Vaduz (LI)
(72) Inventor: Dubowska Inglot, Anna, Prof., PL-53-651 Wroclaw (PL)
(74) Representative: Büchel, Kurt F., Dr.

(56) References cited:
- WO-A-92/16216
- CHEMICAL ABSTRACTS, vol. 101, no. 10, 3 September 1984, Columbus, Ohio, US; abstract no. 78854, TOLPA, STANISLAW ET AL. 'Antineoplastic preparation from the acidified alkaline hydrolysate from peat' page 381 ;
- CHEMICAL ABSTRACTS, vol. 100, no. 17, 23 April 1984, Columbus, Ohio, US; abstract no. 132148, INGLOT, ANNA D. 'Antiviral activity of Wratizolin' page 24
- CHEMICAL ABSTRACTS, vol. 100, no. 17, 23 April 1984, Columbus, Ohio, US; abstract no. 132009, ROMANOWSKA, ELZBIETA ET AL. 'Interaction of Wratizolin with serum albumins and its antiproteolitic activity' page 10 ;
- CHEMICAL ABSTRACTS, vol. 100, no. 22, 28 May 1984, Columbus, Ohio, US; abstract no. 180010, INGLOT, ANNA D. ET AL. 'Biological evaluation of wratizolin penetration from ointments to the skin' page 318 ;

## Description

The present invention relates to bioactive compositions and to pharmaceutical preparations containing same. It is known that certain isothiazole derivatives, particularly amides of 5-aminoisothiazole-4-carboxylic acid, have anti-inflammatory activity comparable with the activity of non-steroidal anti-inflammatory drugs, such as indomethacin. These compounds are also known to possess antiviral, antibacterial and antimycotic activities. A typical representative of these isothiazole derivatives is the p-chlorophenylamide of 3-methyl-5-benzoyl-aminoisothiazole-4-carboxylic acid, also known as Vratizolin^{(R)} (Arch. Immunol. et Ther. Exp. 1973, 21, 891).

On the other hand, peat extracts obtained by various methods using various extraction media are known to contain bioactive ingredients, which render such extracts useful for cosmetic and pharmaceutical purposes. (Chemical Abstracts 101(10), 78854e).

It has now been found that the combination of the above thiazole derivatives with certain peat extracts (peat-derived products) results in an antiviral and anti-inflammatory drug with increased therapeutic potential. Thus, the present invention relates to a bioactive composition which is characterized in that it comprises
a) an isothiazole compound of the general formula I 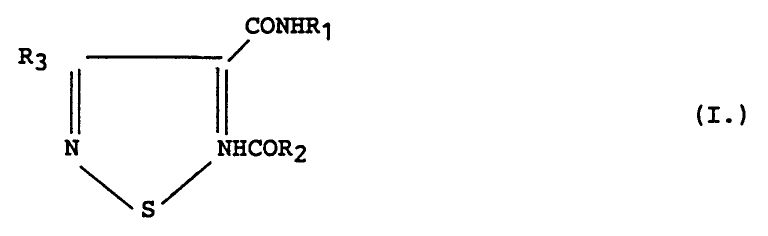 wherein R₁ is halophenyl, R₂ is phenyl and R₃ is lower alkyl, and
b) a peat-derived product containing not more than 70%, preferably not more than 60%, by weight, of inorganic salts, especially of sodium chloride, based on dry solids.

Preferred compositions according to the present invention are characterized in that the isothiazole compound a) is a compound of formula I, wherein R₁ is chlorophenyl, preferably p-chlorophenyl.

With respect to the substituent R₃ of the isothiazole compounds a), those compounds are preferred, wherein R₃ is methyl. The most preferred isothiazole compound a) is a compound of formula I, wherein R₁ is p-chlorophenyl, R₂ is phenyl and R₃ is methyl, i.e. the compound 3-methyl-5-benzoylamino-isothiazole-4-carboxy-p-chloro-phenylamide, also designated as Vratizolin^{(R)}. This compound has the formula II

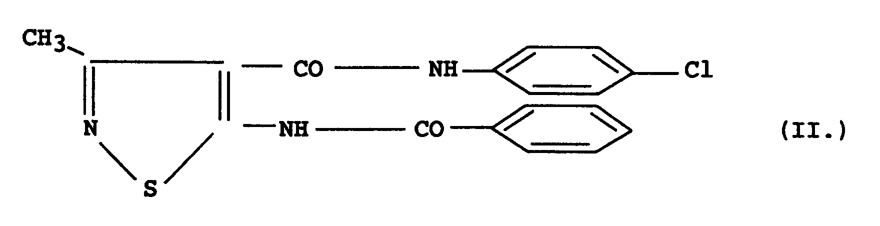

Vratizolin^{(R)} is a registered trade mark in Poland. Further preferred compositions according to the present invention are characterized in that the peat-derived product is obtainable by a process wherein a highly concentrated solution of inorganic salts, especially of sodium chloride, containing peat-derived bioactive ingredients, is diluted with demineralized water and subjected to reverse osmosis in order to desalinate the solution, inorganic salts being removed, and wherein the resulting solution is concentrated and clarified, and, preferably, sterilized and/or spray-dried.

Peat-derived products of this type as well as their production are described in PCT Application No. PCT/EP92/00491 of 4th March, 1992.

A particularly preferred peat-derived product is the product TTP^{(R)} as specified in the above PCT Application and available from Torf Corporation, Wroclaw, ul. Mydlana 2, Poland. It is a spray-dried powder which is easily processable in forming pharmaceutical preparations. TTP^{(R)} stands for "TOLPA^{(R)} Torf Preparation", TOLPA^{(R)} being a registered trade mark in Poland. The weight ratio of compound a) to compound b) in the compositions according to the present invention is about 30:1, preferably 30:2, more preferably 30:3.

The compositions according to the present invention are useful for producing pharmaceutical preparations according to conventional methods by mixing these compositions with pharmaceutically acceptable carriers. Accordingly, the present invention also relates to pharmaceutical preparations comprising a combination of an isothiazole compound as described above and a peat-derived product as described above, together with a pharmaceutically acceptable carrier material.

Such preparations may contain the active ingredient (i.e. the combination of peat-derived product and isothiazole compound) in a quantity of 3.1%, preferably 3.2%, more preferably 3.3% by weight. A preferred pharmaceutical preparation is one which contains Vratizolin^{(R)} and TTP^{(R)}.

Vratizolin^{(R)} and related isothiazoles are not inducers of cytokines, such as interferons (IFN) and tumor necrosis factor (TNF). However, in the presence of IFN and TNF inducers they may stimulate the synthesis of such cytokines and prolong their action.

It was found that the above isothiazole derivatives, such as Vratizolin^{(R)}, administered together with the above peat-derived products, such as TTP^{(R)}, in human peripheral blood leukocyte cultures potentiate the activity of the peat-derived product in the production of IFN and TNF. The combination of the two components a) and b) in the composition according to the present invention was found to be clearly synergistic.

Pharmaceutical preparations according to the present invention, i.e. preparations comprising the components a) and b) are useful for the treatment of herpes virus infections of the skin or mucous membranes (e.g. H. simplex labialis, facialis, H. genitalis, H. zoster varicella, eczema herpetica, other viral infections of mucous membranes, and yarious inflammatory skin diseases.

Pharmaceutical preparations according to the present invention i. e. preparations comprising the components a) and b) are particularly useful for the treatment of genital virus infections transmitted during sexual intercourse and also for the prophylaxis of HIV infections together with condoms.

Examples of pharmaceutical preparations are:
1.

| | |
|---|---|
| Vratizolin^{(R)} | 0.10 |
| TTP^{(R)} | 0.01 |
| Vehiculum | ad 10.00 |

The hydrophilic vehiculum for the treatment of mucous membrane infections is qualitatively composed e.g. of the following ingredients: polyethylene glycol 300, glycerin, polyethylene glycol 4000, and Tween® 80 or ORABASE® Plain of Colgate-Hoyt Labs., Div. of Colgate-Palmolive Comp., One Colgate Way, Canton, WA 02021 USA (ingredients: pectin, sodium carboxymethylcellulose, and plasticized hydrocarbon gel).
The vehiculum for the treatment of skin infections is hydrophilic, with following ingredients: polyethylene glycol 300, glycerin, polyethylene glycol 4000, and Tween 80.
2.

| | |
|---|---|
| Vratizolin^{(R)} | 1.50 |
| TTP^{(R)} | 0.05 |
| Vehiculum | ad 50.00 |

The vehiculum for the treatment and prevention of genital and oral virus infections is as quoted above.

## Claims (Claims for the following Contracting State(s): AT, BE, CH, DE, DK, FR, GB, GR, IE, IT, LI, LU, MC, NL, PT, SE)

1. Bioactive composition, characterized in that it comprises
a)an isothiazole compound of the general formula I 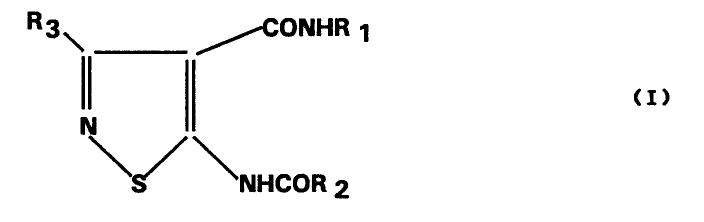 wherein R₁ is halophenyl, R₂ is phenyl and R₃ is lower alkyl and
b) a peat-derived bioactive product containing not more than 70%, preferably not more than 60%, by weight, of inorganic salts, especially of sodium chloride, based on dry solids.

2. A composition as claimed in claim 1, wherein R₁ of compound a) is chlorophenyl, preferably p-chlorophenyl.

3. A composition as claimed in claim 1 or 2, wherein R₃ of compound a) is methyl.

4. A composition as claimed in any one of claims 1 to 3, wherein R₁ of compound a) is p-chlorophenyl, R₂ is phenyl and R₃ is methyl, the compound thus being 3-methyl-5-benzoylaminoisothiazole-4-carboxy-p-chloro-phenylamide (Vratizolin).

5. A composition as claimed in anyone of claims 1 to 4, characterized in that the peat-derived product b) is a product obtainable by a process, wherein a highly concentrated aqueous solution of inorganic salts, especially of sodium chloride, containing peat-derived bioactive ingredients, is diluted with demineralized water and subjected to reverse osmosis in order to desalinate the solution, inorganic salts being removed, and wherein the resulting solution is concentrated and clarified, and, preferably, sterilized and/or spray-dried.

6. A composition as claimed in any one of claims 1 to 5, characterized in that the peat-derived product b) is a bioactive peat extract, preferably in the form of a spray-dried powder.

7. A composition as claimed in anyone of claims 1 to 6, characterized in that the weight ratio of compound a) and product b) is 30:1, preferably 30:2, more preferably 30:3.

8. A composition according to any one of claims 1 to 7, characterized in that it further contains a pharmaceutically acceptable carrier material.

9. A composition as claimed in any one of claims 1 to 8, characterized in that it contains 3-methyl-5-benzoylaminoisothiazole-4-carboxy-p-chloro-phenylamide (Vratizolin) and a bioactive peat extract as defined in claim 6.

10. A composition as claimed in claim 8 or 9, characterized in that it contains compound a) and product b) in a total quantitiy of 3.1%, preferably 3.2%, more preferably 3.3% by weight.

11. A composition according to any one of claims 1 to 10, for use as a medicament.

## Claims (Claims for the following Contracting State(s): ES)

1. Process for the preparation of a bioactive composition, wherein a peat derived bioactive product b) is obtained by a process, in which a highly concentrated aqueous solution of inorganic salts, especially of sodium chloride, containing peat-derived bioactive ingredients, is diluted with demineralized water and subjected to reverse osmosis in order to desalinate the solution, inorganic salts being removed, and wherein the resulting solution is concentrated and clarified, and preferably sterilized and/or spray-dried, characterized in that said peat derived bioactive product b) which contains not more than 70%, preferably not more than 60%, by weight, of inorganic salts, especially of sodium chloride, based on dry solids, is mixed with an isothiazole compound a) of the general formula I 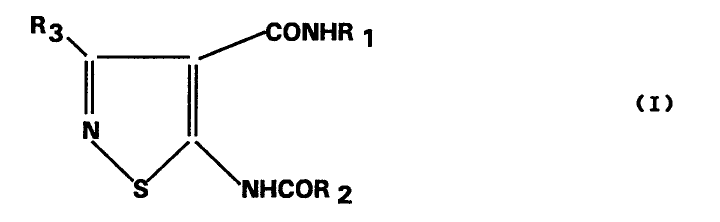 wherein R1 is halophenyl, preferably chlorophenyl, more preferably p-chlorophenyl, R2 is phenyl and R3 is lower alkyl to yield said bioactive composition.

2. Process as claimed in claim 1, wherein R3 of compound a) is methyl.

3. Process as claimed in claim 1 or 2, wherein R1 of compound a) is p-chlorophenyl, R2 is phenyl and R3 is methyl, the compound thus being 3-methyl-5-benzoylaminoisothiazole-4-carboxy-p-chloro-phenylamide (Vratizolin).

4. Process as claimed in any one of claims 1 to 3, characterized in that a bioactive peat extract, preferably in the form of a spray-dried powder, is used as said peat-derived product.

5. Process as claimed in any one of claims 1 to 4, characterized in that the weight ratio of compound a) and peat derived product b) is 30:1, preferably 30:2, more preferably 30:3.

6. Process as claimed in any one of the preceding claims, characterized in that the bioactive composition is prepared by mixing 3-methyl-5-benzoylaminoisothiazole-4-carboxy-p-chloro-phenylamide (Vratizolin) and a bioactive peat extract obtainable by a process as defined in claim 4.

7. Process as claimed in any one of claims 1 to 6, characterized in that compound a) and peat derived product b) are further mixed with a pharmaceutically acceptable carrier material.

8. Process as claimed in claim 6 or 7, characterized in that the composition contains compound a) and product b) in a total quantity of 3,1%, preferably 3,2%, more preferably 3,3% by weight.

9. Bioactive composition, characterized in that it comprises
a)an isothiazole compound of the general formula I 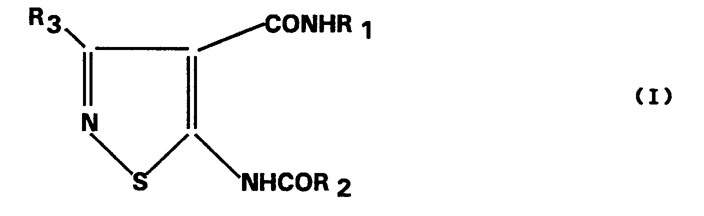 wherein R₁ is halophenyl, R₂ is phenyl and R₃ is lower alkyl and
b) a peat-derived bioactive product containing not more than 70%, preferably not more than 60%, by weight, of inorganic salts, especially of sodium chloride, based on dry solids.

10. A composition as claimed in claim 9, wherein R₁ of compound a) is chlorophenyl, preferably p-chlorophenyl.

11. A composition as claimed in claim 9 or 10, wherein R₃ of compound a) is methyl.

12. A composition as claimed in any one of claims 9 to 11, wherein R₁ of compound a) is p-chlorophenyl, R₂ is phenyl and R₃ is methyl, the compound thus being 3-methyl-5-benzoylaminoisothiazole-4-carboxy-p-chloro-phenylamide (Vratizolin).

13. A composition as claimed in anyone of claims 9 to 12, characterized in that the peat-derived product b) is a product obtainable by a process, wherein a highly concentrated aqueous solution of inorganic salts, especially of sodium chloride, containing peat-derived bioactive ingredients, is diluted with demineralized water and subjected to reverse osmosis in order to desalinate the solution, inorganic salts being removed, and wherein the resulting solution is concentrated and clarified, and, preferably, sterilized and/or spray-dried.

14. A composition as claimed in any one of claims 9 to 13, characterized in that the peat-derived product b) is a bioactive peat extract, preferably in the form of a spray-dried powder.

15. A composition as claimed in anyone of claims 9 to 14, characterized in that the weight ratio of compound a) and product b) is 30:1, preferably 30:2, more preferably 30:3.

16. A composition according to any one of claims 9 to 15, characterized in that it contains compound a) and product b) together with a pharmaceutically acceptable carrier material.

17. A composition as claimed in any one of claims 9 to 16, characterized in that it contains 3-methyl-5-benzoylaminoisothiazole-4-carboxy-p-chloro-phenylamide (Vratizolin) and a bioactive peat extract as defined in claim 14.

18. A composition as claimed in claim 16 or 17, characterized in that it contains compound a) and product b) in a total quantitiy of 3.1%, preferably 3.2%, more preferably 3.3% by weight.

19. A composition according to any one of claims 9 to 18 or manufactured according to any one of claims 1 to 8, for use as a medicament.

## Patentansprüche (Patentansprüche für folgende(n) Vertragsstaat(en): AT, BE, CH, DE, DK, FR, GB, GR, IE, IT, LI, LU, MC, NL, PT, SE)

1. Bioaktive Zusammensetzung, dadurch gekennzeichnet, daß sie aufweist:
a) eine Isothiazolverbindung der allgemeinen Formel I 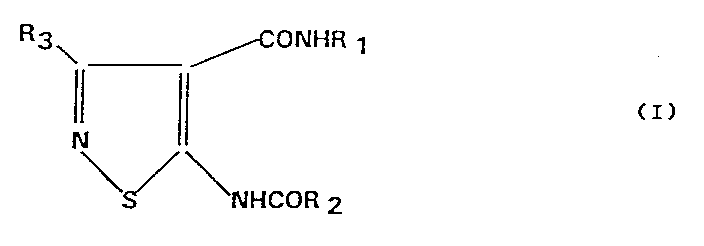 worin R₁ ein Halogenphenyl, R₂ ein Phenyl und R₃ ein niedriges Alkyl ist und
b) ein von Torf abgeleitetes Produkt, welches nicht mehr als 70 Gewichts-%, vorzugsweise nicht mehr als 60%, anorganischer Salze, insbesondere Natriumchlorid, basierend auf den trockenen Feststoffen, enthält.

2. Zusammensetzung nach Anspruch 1, bei der R₁ der Verbindung a) Chlorphenyl, vorzugsweise p-Chlorphenyl, ist.

3. Zusammensetzung nach Anspruch 1 oder 2, bei der R₃ der Verbindung a) Methyl ist.

4. Zusammensetzung nach einem der Ansprüche 1 bis 3, bei der R₁ der Verbindung a) p-Chlorphenyl, R₂ Phenyl und R₃ Methyl ist, womit die Verbindung 3-Methyl-5-Benzoylaminoisothiazol-4-Carboxy-p-Chlor-Phenylamid (Vratizolin) ist.

5. Zusammensetzung nach einem der Ansprüche 1 bis 4, dadurch gekennzeichnet, daß das von Torf abgeleitete Produkt b) ein Produkt ist, das durch ein Verfahren erhältlich ist, bei dem eine hochkonzentrierte wäßrige Lösung anorganischer Salze, insbesondere von Natriumchlorid, welche von Torf abgeleitete bioaktive Ingredienzien enthält, mit entmineralisiertem Wasser verdünnt und einer Umkehrosmose unterworfen wird, um die Lösung zu entsalzen, wobei anorganische Salze entfernt werden, und wobei die sich ergebende Lösung konzentriert und geklärt, und vorzugsweise sterilisiert und/oder sprühgetrocknet, wird.

6. Zusammensetzung nach einem der Ansprüche 1 bis 5, dadurch gekennzeichnet, daß das von Torf abgeleitete Produkt b) ein bioaktiver Torfextrakt, vorzugsweise in Form eines sprühgetrockneten Pulvers, ist.

7. Zusammensetzung nach einem der Ansprüche 1 bis 6, dadurch gekennzeichnet, daß das Gewichtsverhältnis der Verbindung a) und des Produktes b) 30:1, vorzugsweise 30:2, bevorzugter 30:3, beträgt.

8. Zusammensetzung nach einem der Ansprüche 1 bis 7, dadurch gekennzeichnet, daß sie ferner ein pharmazeutisch akzeptables Trägermaterial enthält.

9. Zusammensetzung nach einem der Ansprüche 1 bis 8, dadurch gekennzeichnet, daß sie 3-Methyl-5-Benzoylaminoisothiazol-4-Carboxy-p-Chlor-Phenylamid (Vratizolin) und einen bioaktiven Torfextrakt enthalt, wie er in Anspruch 6 definiert ist.

10. Zusammensetzung nach Anspruch 8 oder 9, dadurch gekennzeichnet, daß sie die Verbindung a) und das Produkt b) in einer Gesamtmenge von 3,1 Gewichts-%, vorzugsweise 3,2%, bevorzugter 3,3%, enthalt.

11. Zusammensetzung nach einem der Ansprüche 1 bis 10 für die Verwendung als Medikament.

## Patentansprüche (Patentansprüche für folgende(n) Vertragsstaat(en): ES)

1. Verfahren für die Herstellung einer bioaktiven Zusammensetzung, bei dem ein von Torf abgeleitetes Produkt b) durch ein Verfahren erhalten wird, bei dem eine hochkonzentrierte wäßrige Lösung anorganischer Salze, insbesondere von Natriumchlorid, welche von Torf abgeleitete bioaktive Ingredienzien enthält, mit entmineralisiertem Wasser verdünnt und einer Umkehrosmose unterworfen wird, um die Lösung zu entsalzen, wobei anorganische Salze entfernt werden, und wobei die sich ergebende Lösung konzentriert und geklärt, und vorzugsweise sterilisiert und/oder sprühgetrocknet, wird, dadurch gekennzeichnet, daß das von Torf abgeleitete Produkt b), welches nicht mehr als 70 Gewichts-%, vorzugsweise nicht mehr als 60%, anorganischer Salze, insbesondere Natriumchlorid, basierend auf den trockenen Feststoffen, enthält, mit einer Isothiazolverbindung a) der allgemeinen Formel I 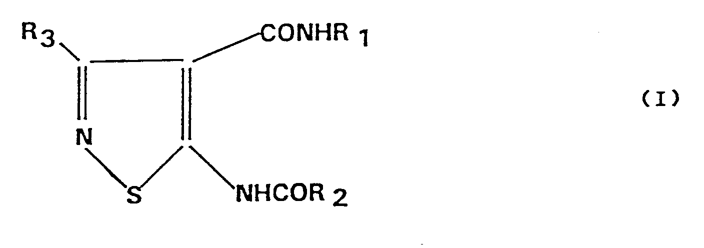 gemischt wird, worin R₁ ein Halogenphenyl, vorzugsweise Chlorphenyl, vorzugsweise p-Chlorphenyl, ist, R₂ ein Phenyl und R₃ ein niedriges Alkyl ist,
um die bioaktive Zusammensetzung zu gewinnen.

2. Verfahren nach Anspruch 1, bei der R₃ der Verbindung a) Methyl ist.

3. Verfahren nach Anspruch 1 oder 2, bei dem R₁ der Verbindung a) p-Chlorphenyl, R₂ Phenyl und R₃ Methyl ist, womit die Verbindung 3-Methyl-5-Benzoylaminoisothiazol-4-Carboxy-p-Chlor-Phenylamid (Vratizolin) ist.

4. Verfahren nach einem der Ansprüche 1 bis 3, dadurch gekennzeichnet, daß ein bioaktiver Torfextrakt, vorzugsweise in Form eines sprühgetrockneten Pulvers, als das von Torf abgeleitete Produkt verwendet wird.

5. Verfahren nach einem der Ansprüche 1 bis 4, dadurch gekennzeichnet, daß das Gewichtsverhältnis der Verbindung a) und des von Torf abgeleiteten Produktes b) 30:1, vorzugsweise 30:2, bevorzugter 30:3, beträgt.

6. Verfahren nach einem der vorhergehenden Ansprüche, dadurch gekennzeichnet, daß die bioaktive Zusammensetzung durch Mischen von 3-Methyl-5-Benzoylaminoisothiazol-4-Carboxy-p-Chlor-Phenylamid (Vratizolin) und eines bioaktiven Torfextraktes hergestellt wird, der durch ein Verfahren erhältlich ist, wie es in Anspruch 4 definiert ist.

7. Verfahren nach einem der Ansprüche 1 bis 6, dadurch gekennzeichnet, daß die Verbindung a) und das von Torf abgeleitete Produkt b) ferner mit einem pharmazeutisch akzeptablen Trägermaterial gemischt werden.

8. Verfahren nach Anspruch 6 oder 7, dadurch gekennzeichnet, daß die Zusammensetzung die Verbindung a) und das Produkt b) in einer Gesamtmenge von 3,1 Gewichts-%, vorzugsweise 3,2%, bevorzugter 3,3%, enthalt.

9. Bioaktive Zusammensetzung, dadurch gekennzeichnet, daß sie aufweist:
a) eine Isothiazolverbindung der allgemeinen Formel I 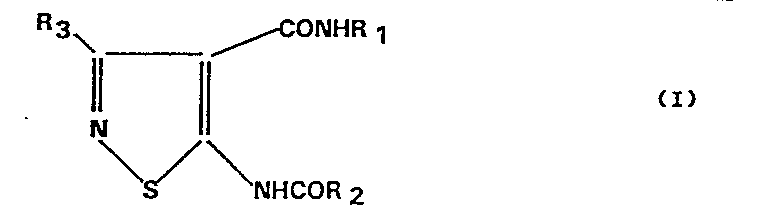 worin R₁ ein Halogenphenyl, R₂ ein Phenyl und R₃ ein niedriges Alkyl ist und
b) ein von Torf abgeleitetes bioaktives Produkt, welches nicht mehr als 70 Gewichts-%, vorzugsweise nicht mehr als 60%, anorganischer Salze, insbesondere Natriumchlorid, basierend auf den trockenen Feststoffen, enthält.

10. Zusammensetzung nach Anspruch 9, bei der R₁ der Verbindung a) Chlorphenyl, vorzugsweise p-Chlorphenyl, ist.

11. Zusammensetzung nach Anspruch 9 oder 10, bei der R₃ der Verbindung a) Methyl ist.

12. Zusammensetzung nach einem der Ansprüche 9 bis 11, bei der R₁ der Verbindung a) p-Chlorphenyl, R₂ Phenyl und R₃ Methyl ist, womit die Verbindung 3-Methyl-5-Benzoylaminoisothiazol-4-Carboxy-p-Chlor-Phenylamid (Vratizolin) ist.

13. Zusammensetzung nach einem der Ansprüche 9 bis 12, dadurch gekennzeichnet, daß das von Torf abgeleitete Produkt b) ein Produkt ist, das durch ein Verfahren erhältlich ist, bei dem eine hochkonzentrierte wäßrige Lösung anorganischer Salze, insbesondere von Natriumchlorid, welche von Torf abgeleitete bioaktive Ingredienzien enthält, mit entmineralisiertem Wasser verdünnt und einer Umkehrosmose unterworfen wird, um die Lösung zu entsalzen, wobei anorganische Salze entfernt werden, und wobei die sich ergebende Lösung konzentriert und geklärt, und vorzugsweise sterilisiert und/oder sprühgetrocknet, wird.

14. Zusammensetzung nach einem der Ansprüche 9 bis 13, dadurch gekennzeichnet, daß das von Torf abgeleitete Produkt b) ein bioaktiver Torfextrakt, vorzugsweise in Form eines sprühgetrockneten Pulvers, ist.

15. Zusammensetzung nach einem der Ansprüche 9 bis 14, dadurch gekennzeichnet, daß das Gewichtsverhältnis der Verbindung a) und des Produktes b) 30:1, vorzugsweise 30:2, bevorzugter 30:3, beträgt.

16. Zusammensetzung nach einem der Ansprüche 9 bis 15, dadurch gekennzeichnet, daß sie die Verbindung a) und das Produkt b) zusammen mit einem pharmazeutisch akzeptablen Trägermaterial enthält.

17. Zusammensetzung nach einem der Ansprüche 9 bis 16, dadurch gekennzeichnet, daß sie 3-Methyl-5-Benzoylaminoisothiazol-4-Carboxy-p-Chlor-Phenylamid (Vratizolin) und einen bioaktiven Torfextrakt enthält, wie er in Anspruch 14 definiert ist.

18. Zusammensetzung nach Anspruch 16 oder 17, dadurch gekennzeichnet, daß sie die Verbindung a) und das Produkt b) in einer Gesamtmenge von 3,1 Gewichts-%, vorzugsweise 3,2%, bevorzugter 3,3%, enthalt.

19. Zusammensetzung nach einem der Ansprüche 9 bis 18 bzw. welche nach einem der Ansprüche 1 bis 8 hergestellt ist, für die Verwendung als Medikament.

## Revendications (Revendications pour l'(les) Etat(s) contractant(s) suivant(s): AT, BE, CH, DE, DK, FR, GB, GR, IE, IT, LI, LU, MC, NL, PT, SE)

1. Composition bioactive, caractérisée en ce qu'elle comprend
a) un composé d'isothiazole de formule générale I 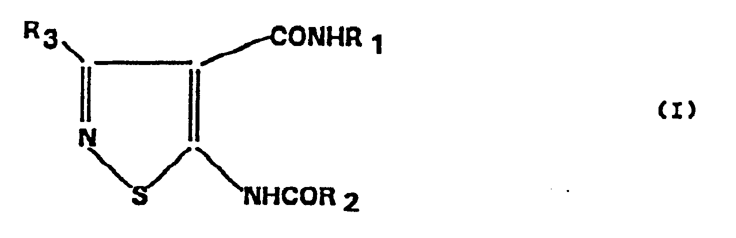 dans laquelle R₁ est un halophényle, R₂ est un phényle et R₃ est un alkyle inférieur, et
b) un produit bioactif dérivé de tourbe, ne contenant pas plus de 70 %, de préférence pas plus de 60 % en poids, de sels inorganiques, spécialement de chlorure de sodium, basé sur la teneur en solides secs.

2. Une composition selon la revendication 1, dans laquelle R₁ du composé a) est du chlorophényle, de préférence du p-chlorophényle.

3. Une composition selon la revendication 1 ou 2, dans laquelle R₃ du composé a) est du méthyle.

4. Une composition selon l'une quelconque des revendications 1 à 3, dans laquelle R₁ est un composé a) est du p-chlorophényle, R₂ est du phényle et R₃ est du méthyle, le composé ainsi étant du 3-méthyl-5-benzoylaminoisothiazole-4-carboxy-p-chloro-phénylamide (Vratizoline).

5. Une composition selon l'une quelconque des revendications 1 à 4, caractérisée en ce que le produit b) dérivé de la tourbe est un produit pouvant être obtenu par un procédé dans lequel une solution aqueuse, fortement concentrée, de sels inorganiques, spécialement de chlorure de sodium, contenant des ingrédients bioactifs dérivés de la tourbe, est diluée avec de l'eau déminéralisée et soumise à une osmose inverse pour dessaler la solution, les sels inorganiques étant éliminés, et dans laquelle la solution résultante est concentrée et est clarifiée et, de préférence, stérilisée et/ou séchée par pulvérisation.

6. Une composition selon l'une quelconque des revendications 1 à 5, caractérisée en ce que le produit dérivé de la tourbe b) est un extrait de tourbe bioactif, de préférence se présentant sous la forme d'une poudre séchée par pulvérisation.

7. Une composition selon l'une quelconque des revendications 1 à 6, caractérisée en ce que leur rapport en poids entre le composé a) et le produit b) est de 30:1, de préférence de 30:2, de manière mieux préférée de 30:3.

8. Une composition selon l'une quelconque des revendications 1 à 7, caractérisée en ce qu'elle contient en outre un matériau support pharmaceutiquement acceptable.

9. Une composition selon l'une quelconque des revendications 1 à 8, caractérisée en ce qu'elle contient du 3-méthyl-5-benzoylaminoisothiazole-4-carboxy-p-chlorophénylamide (Vratizoline) et un extrait de tourbe bioactif tel que défini à la revendication 6.

10. Une composition selon la revendication 8 ou 9, caractérisée en ce qu'elle contient le composé a) et le produit b) en une quantité totale de 3,1 % en poids, de préférence de 3,2 % en poids, de manière mieux préférée de 3,3 % en poids.

11. Une composition selon l'une quelconque des revendications 1 à 10, destinée à être utilisée comme médicament.

## Revendications (Revendications pour l'(les) Etat(s) contractant(s) suivant(s): ES)

1. Procédé de préparation d'une composition bioactive, dans lequel un produit bioactif b) dérivé de la tourbe est obtenu par un procédé dans lequel une solution aqueuse, fortement concentrée, de sels inorganiques, spécialement de chlorure de sodium, contenant des ingrédients bioactifs dérivés de la tourbe, est diluée avec de l'eau déminéralisée et soumise à une osmose inverse pour dessaler la solution, les sels inorganiques étant éliminés, et dans lequel la solution résultante est concentrée et est clarifiée et, de préférence, stérilisée et/ou séchée par pulvérisation, caractérisé en ce que ledit produit bioactif b) dérivé de la tourbe, ne contenant pas plus de 70 %, de préférence pas plus de 60 % en poids de sels inorganiques, spécialement de chlorure de sodium, basé sur la teneur en solides secs, est mélangé à de l'isothiazole à titre de composé a) de la formule générale I 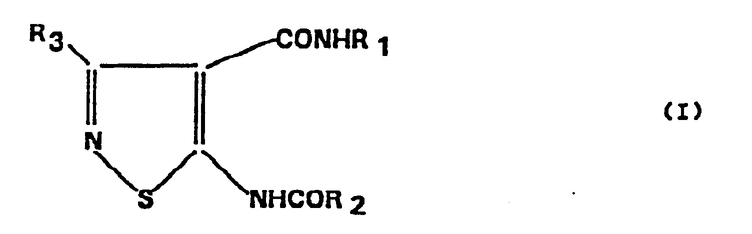 dans laquelle R₁ est de l'halophényle, de préférence du chlorophényle, de manière mieux préférée du p-chlorophényle, R₂ est du phényle et R₃ est un alkyle inférieur,
afin de produire ladite composition bioactive.

2. Procédé selon la revendication 1, dans lequel R₃ du composé a) est du méthyle.

3. Procédé selon la revendication 1 ou 2, dans lequel R₁ du composé a) est du p-chlorophényle, R₂ est du phényle et R₃ est du méthyle, le composé ainsi, étant du 3-méthyl-5-benzoylaminoisothiazole-4-carboxy-p-chlorophénylamide (Vratizoline).

4. Procédé selon l'une quelconque des revendications 1 à 3, caractérisé en ce qu'un extrait de tourbe bioactif, de préférence se présentant sous la forme d'une poudre séchée par pulvérisation, est utilisé comme dit produit dérivé de la tourbe.

5. Procédé selon l'une quelconque des revendications 1 à 4, caractérisé en ce que le rapport en poids entre le composant a) et le produit b) dérivé de la tourbe est de 30:1, de préférence 30:2, de préférence 30:3.

6. Procédé selon l'une quelconque des revendications précédentes, caractérisé en ce que la composition bioactive est préparée par mélange de 3-méthyl-5-benzoylaminoisothiazole-4-carboxy-p-chloro-phénylamide (Vratizoline) et d'un extrait de tourbe bioactif pouvant être obtenu par un procédé tel que défini dans la revendication 4.

7. Procédé selon l'une quelconque des revendications 1 à 6, caractérisée en ce que le composant a) et le produit b) dérivé de la tourbe sont en outre mélangés à un matériau support pharmaceutiquement acceptable.

8. Procédé selon la revendication 6 ou 7, caractérisé en ce que la composition contient le composé a) et le produit b) en une quantité totale de 3,1 % en poids, de préférence de 3,2 % en poids, de manière mieux préférée de 3,3 % en poids.

9. Composition bioactive, caractérisée en ce qu'elle comprend
a) un composé isothiazole de formule générale I 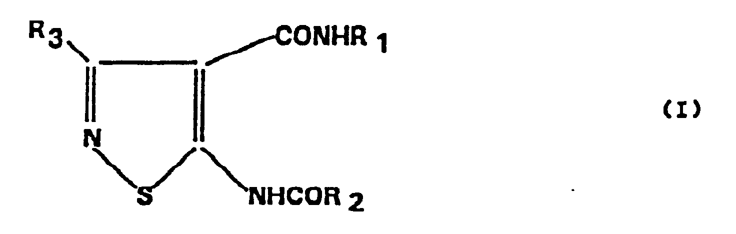 dans laquelle R₁ est de l'halophényle, R₂ est du phényle et R₃ est un alkyle inférieur, et
b) un produit bioactif dérivé de tourbe ne contenant pas plus de 70 %, de préférence pas plus de 60 % en poids, de sels inorganiques, spécialement de chlorure de sodium, basé sur la teneur en solides secs.

10. Une composition selon la revendication 9, dans laquelle R₁ du composé a) est du chlorophényle, de préférence du p-chlorophényle.

11. Un composé selon la revendication 9 ou 10, dans lequel R3 du composé a) est du méthyle.

12. Un composé selon l'une quelconque des revendications 9 à 11, dans lequel R₁ du composé a) est du p-chlorophényle, R₂ est du phényle et R₃ est du méthyle, le composé ainsi étant du 3-méthyl-5-benzoylaminoisothiazole-4-carboxy-p-phénylamide (Vratizoline).

13. Une composition selon l'une quelconque des revendication 9 à 12, caractérisée en ce que le produit dérivé b) de la tourbe est un produit pouvant être obtenu par un procédé dans lequel une solution aqueuse, fortement concentrée, de sels inorganiques, spécialement de chlorure de sodium, contenant des ingrédients bioactifs dérivés de la tourbe, est diluée avec de l'eau déminéralisée et soumise à une osmose inverse pour dessaler la solution, les sels inorganiques étant éliminés, et dans laquelle la solution résultante est concentrée et est clarifiée et, de préférence, stérilisée et/ou séchée par pulvérisation.

14. Une composition selon l'une quelconque des revendication 9 à 13, caractérisée en ce que le produit dérivé de la tourbe b) est un extrait de tourbe bioactif, de préférence se présentant sous la forme d'une poudre séchée par pulvérisation.

15. Une composition selon l'une quelconque des revendication 9 à 14, caractérisée en ce que le rapport en poids entre le composé a) et le produit b) est de 30:1, de préférence de 30:2, de manière mieux préférée de 30:3.

16. Une composition selon l'une quelconque des revendications 9 à 15, caractérisée en ce qu'elle contient le composé a) et le produit b), conjointement avec un matériau support pharmaceutiquement acceptable.

17. Une composition selon l'une quelconque des revendications 9 à 16, caractérisée en ce qu'elle contient du 3-méthyl-5-benzoylaminoisothiazole-4-carboxy-p-chlorophénylamide (Vratizoline) et un extrait de tourbe bioactif tel que défini à la revendication 14.

18. Une composition selon la revendication 16 ou 17, caractérisée en ce qu'elle contient le composé a) et le produit b) en une quantité totale de 3,1 % en poids, de préférence de 3,2 % en poids, de manière mieux préférée de 3,3 % en poids.

19. Une composition selon l'une quelconque des revendications 9 à 18 ou fabriquée selon l'une quelconque des revendications 1 à 8, pour utilisation à titre de médicament.
